# EUROPEAN PATENT SPECIFICATION

(11) **EP 3 645 506 B1**
(45) Date of publication and mention of the grant of the patent: **24.11.2021**
(21) Application number: 18748949.7
(22) Date of filing: 27.06.2018
(51) Int. Cl.: C07C 227/08, C07C 227/16, D21C 9/10, C07C 229/12, C07C 229/24

(54) **PROCESS FOR THE PREPARATION OF A MIXTURE OF CHELATING AGENTS, MIXTURE OF CHELATING AGENTS AND METHODS OF USING THEM**
VERFAHREN ZUR HERSTELLUNG EINER MISCHUNG AUS CHELATBILDNERN, MISCHUNG AUS CHELATBILDNERN UND VERFAHREN ZUR VERWENDUNG DAVON
PROCÉDÉ DE PRÉPARATION D'UN MÉLANGE D'AGENTS CHÉLATANTS, MÉLANGE D'AGENTS CHÉLATANTS ET PROCÉDÉS D'UTILISATION DE CEUX-CI

(30) Priority: 30.06.2017 FI 20175631
(43) Date of publication of application: 06.05.2020
(73) Proprietor: Kemira Oyj, 00180 Helsinki (FI)
(72) Inventor: AKSELA, Reijo, 02360 Espoo (FI); KONN, Jonas, 02270 Espoo (FI); PERANDER, Anna-Maija, 02270 Espoo (FI); HAARALA, Anna, 02270 Espoo (FI); ILMONIEMI, Riitta, 02400 Kirkkonummi (FI); HOFFREN, Hanna, 00980 Helsinki (FI)
(74) Representative: Berggren Oy
(86) International application number: PCT/FI2018/050504
(87) International publication number: WO 2019/002684

(56) References cited:
- WO-A1-97/45396
- WO-A1-99/46234

## Description

The present invention relates to a process for in situ the preparation of mixtures of chelating agents by catalyzed reactions of diethanolamine with maleic acid and then with 2-halocarboxylic acid, mixtures obtainable using said process and mixtures of chelating agents. In addition, the invention relates to methods where such mixtures are used.

### Background

In pulp bleaching liquors, iron and manganese are desired to be trapped by a chelating agent, thereby inhibiting these metal ions from catalyzing the decomposition of the bleaching agents, hydrogen peroxide or peroxy acids. Because there is naturally a high concentration of calcium ions in the bleaching liquors, a chelating agent effectively chelating calcium would be consumed by calcium ions. Therefore, chelating agents to selectively complex iron and manganese ions are desired.

WO 97/45396 discloses N-bis- and N-tris-[(1,2-dicarboxy-ethoxy)-ethyl]-amine derivatives including N-bis-[(1,2-dicarboxy-ethoxy)-ethyl]-aspartic acid (also called aspartic acid diethoxy succinate or AES), and the use of these derivatives as chelating agents of metals, especially in connection with pulp bleaching. These derivatives can be prepared by reacting di- or triethanolamine with an alkali metal or alkaline earth metal salt of maleic acid in the presence of a catalyst such as lanthanoid compounds, a nickel compound or alkaline earth metal compounds, e.g. calcium hydroxide or magnesium hydroxide.

WO 99/46234 discloses a method for the preparation of an N-bis-[2-(1,2-dicar-boxy-ethoxy)-ethyl]-amine derivative. In said method an alkali or earth alkali metal salt of maleic acid is brought to a reaction with an N-substituted diethanolamine.

A drawback with the above-mentioned synthesis of e.g. AES is that the reaction is relatively slow, the reaction time being about 12 to 16 hours, and that the reaction does not go to completion. A typical obtainable conversion from diethanol amine to AES products is about 60 to 70%. This means that a significant amount, up to about 40 mol-%, of the diethanol amine (DEA) used as a starting material is left unreacted.

In order to simultaneously complex different metal ions in aqueous solutions, it is essential in many applications to have a mixture of chelating agents having different ligand structure. Therefore, there is a need to develop a method for the preparation of mixtures of chelating agents *in situ* in the same reaction mixture. In addition, there is a continuous need for a process where the starting materials are efficiently converted into chelating agents.

### Summary of the invention

According to the present invention it was surprisingly found that the unreacted diethanolamine can easily and effectively be converted into other reactive ingredients by the addition of a 2- haloalkyl carboxylic acid compound into the reaction containing a lanthanoid catalyst to yield an amino acid derivative, thereby utilizing the unreacted diethanol amine.

It has now been surprisingly found that mixtures of chelating agents having a diethanol amine backbone can be effectively prepared by lanthanoid catalysed reaction of diethanol amine with maleate by a lanthanoid-catalyzed reaction of 2-halocarboxylic acid. Alternatively, mixtures of chelating agents can effectively be prepared by a lanthanoid catalyzed reaction of diethanol amine with 2-halocarboxylic acid followed by addition of maleate.

The first aspect of the present invention is a process for the preparation of a mixture of chelating agents comprising a compound of Formula (I) 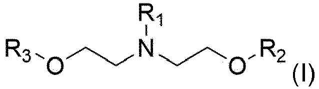 wherein at least one of R₁, R₂ and R₃ is a succinic acid group or a salt thereof and at least one of R₁, R₂ and R₃ is a carboxymethyl or 1-carboxyethyl group or a salt thereof. According to the invention said reaction comprises reacting maleic acid or a salt thereof with diethanol amine under alkaline conditions in the presence of a lanthanoid catalyst to form at least one compound having a general formula (I) where at least one of R1, R2 and R3 is a succinic acid group or a salt thereof followed by adding of 2-halocarboxylic acid or a salt thereof which reacts with unreacted diethanol amine and/or with intermediates containing hydroxyl groups or secondary amino groups to form a mixture comprising compounds having a general formula (I) where at least one of R₁, R₂ and R₃ is succinic acid group or a salt thereof and at least one of R₁, R₂ and R₃ is carboxymethyl or 1-carboxyethyl group or a salt thereof.

The second aspect of this invention is a mixture of chelating agents. According to the invention said mixture comprises at least 30 % (w/w) of AES6 and at least 2 % (w/w) of AES5 or GES5 or combination of AES5 and GES5 (N-[2-(1,2-dicarboxyethoxy)ethyl]-N-[2-(carboxymethoxy)ethyl]aspartic acid, AES5; glycine diethoxy succinate, GES5; aspartic acid diethyl succinate, AES6).

The third aspect of this invention is a method of chelating metals by contacting the mixture here described with aqueous slurry comprising the metals.

The fourth aspect of this invention is a method of bleaching pulp comprising treating the pulp with the mixture described here or adding the mixture described here to the bleaching stage.

### Brief description of figure

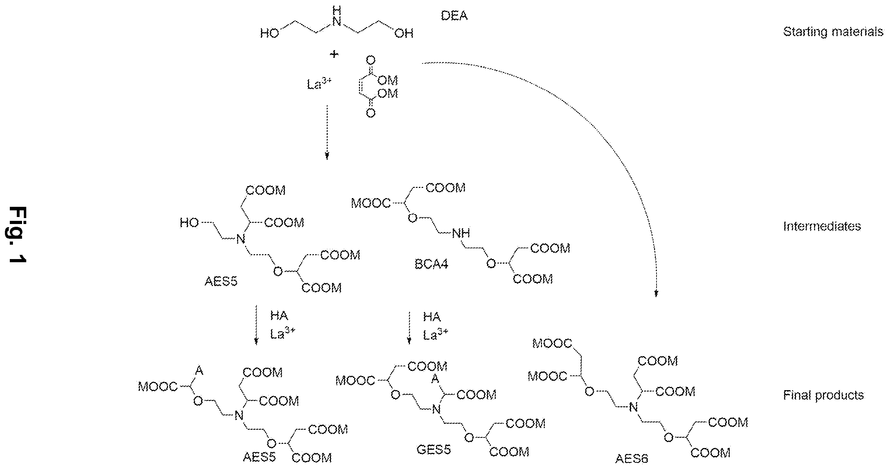

Figure 1 shows a simplified reaction scheme for one possible embodiment of the present invention. In figure 1, A is H or methyl, and M is H or a metal ion. HA is a 2-halocarboxylic acid.

### Detailed description

As used here expression "a mixture of chelating agents" means a mixture comprising at least two differently substituted chelating agents synthesized starting from diethanolamine.

Maleate as used herein means maleic acid or a salt thereof.

As used herein the expression intermediate(s) means compounds having a diethanolamine backbone and a general formula (I) where at least one of R₁, R₂ and R₃ is a succinic acid group, carboxymethyl or carboxyethyl group, and the compound contains at least one unreacted/free hydroxyl group or a secondary amino group.

As used herein the expression succinic acid group means a substituent formed in the Michael-addition of a maleate to a hydroxyl group or the hydroamination reaction of a maleate and a secondary amine including both carboxylic acids and salts thereof.

As used herein the term 2-halocarboxylic acid means saturated carboxylic acids substituted with a halogen atom in the 2-position. The carboxylic acid is preferably acetic acid or propionic acid. The halogen is preferably bromine or chlorine.

As used herein the term equilibrium means an ordinary chemical equilibrium of a reaction.

A method for the preparation of mixtures of chelating agents *in situ* is described here. This method accomplishes the conversion of most of the starting materials to chelating agents. A mixture comprising chelating agents with different capability to complex metal cations is many times advantageous for a complete deactivation of the metal ions in for example pulp bleaching applications. In addition, it is economically and environmentally advantageous to have the starting materials of the chelating agent synthesis reacted as completely as possible to form useful reaction products. In addition, the mixture comprising products obtained using the method here described is essentially free of diethanol amine that could form harmful or toxic nitrosamines. The composition of the metal complexing molecules can be easily adjusted by varying the ratios of the reagents used in the synthesis of the mixtures of chelating agents.

The metal complexing ability of mixtures of chelating agents is usually better than the complexing ability of individual chelating agents. This is especially noticed in the pulp bleaching applications, where iron, manganese, calcium and magnesium ions are present in the bleaching liquor.

Compared to traditionally used chelating agents such as ethylenediamine tetraacetic acid (EDTA) and diethylenetriamine pentaacetic acid (DTPA), the chelating agents prepared by the method described here, are more biodegradable. Some of the compounds obtained by method described here are readily biodegradable (e.g. ethylenedisuccinic acid EDDS and iminodisuccinic acid IDS) and generally the diethanolamine originated polycarboxylic acids are at least inherently biodegradable.

An aspect of the present invention is the process for preparation of a mixture of chelating agents comprising a compound of Formula (I) 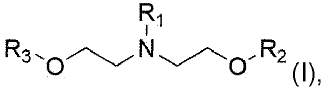 wherein at least one of R₁, R₂ and R₃ is a succinic acid group or a salt thereof and at least one of R₁, R₂ and R₃ is a carboxymethyl or 1-carboxyethyl group or a salt thereof. Said process comprises reacting maleic acid or a salt thereof with diethanol amine under alkaline conditions in the presence of a lanthanoid catalyst to form at least one compound having a general formula (I) wherein at least one of R₁, R₂ and R₃ is a succinic acid group or a salt thereof followed by addition of 2-halocarboxylic acid or a salt thereof which reacts with unreacted diethanol amine and/or with intermediates containing hydroxyl groups or secondary amino groups to form a mixture comprising compounds having a general formula (I) wherein at least one of R₁, R₂ and R₃ is a succinic acid group or a salt thereof and at least one of R₁, R₂ and R₃ is a carboxymethyl or 1-carboxyethyl group or a salt thereof.

In this connection expression "carboxymethyl or 1-carboxyethyl group or a salt thereof" means a monocarboxylic acid group (or a salt thereof) derived from the reaction of a hydroxyl group or a secondary amine with a 2-haloalkylcarboxylic acid or a salt thereof.

In one embodiment unreacted diethanol amine (in practice diethanol amine that has not reacted with maleic acid in the first reaction step), is N-alkylated with a halocarboxylic acid to form a tertiary amine group, such as bis-(2-hydroxyethyl)glycine or bis-(2-hydroxyethyl)methyl glycine. Such reaction catalyzed as described here provides a reaction mixture essentially free of diethanol amine. Unreacted diethanol amine could form harmful nitrosamines.

In one embodiment the reaction is continued until the reaction mixture comprises at least one compound of Formula (I) wherein at least one of R₁, R₂ and R₃ is a carboxymethyl or 1-carboxyethyl group or a salt thereof and wherein the two remaining of R₁, R₂ and R₃ are succinic acid groups or salts thereof (AES5 or GES5), and at least one compound with Formula (I) wherein R₁, R₂ and R₃ are succinic acid groups (AES6), and bis-(2-hydroxyethyl)glycine or bis-(2-hydroxyethyl)methyl glycine.

2-halocarboxylic acid as used herein includes 2-halocarboxylic acids as acids and salts. Said acid can be any carboxylic acid containing 2 to 3 carbon atoms, typically haloacetic acid or 2-halopropionic acid. The halogen is usually bromine or chlorine, the latter being preferred for environmental reasons.

One possible simplified reaction scheme is illustrated in Figure 1. In Figure 1, A is H or methyl, and M is H or a metal ion. HA is a 2-halocarboxylic acid.

In one embodiment the 2-halocarboxylic acid is bromo- or chloroacetic acid, preferably 2-chloroacetic acid. In another embodiment the 2-halocarboxylic acid can be 2-chloro- or 2-bromopropionic acid, preferably 2-chloropropionic acid.

Chlorine-containing starting materials are preferred due to the difficulties in recycling the hydrobromic acid formed in the reaction. Furthermore, colored bromine-containing side-products which are undesirable in pulping applications are formed.

The non-catalyzed reaction of 2-halocarboxylic acids with an amino group is known in the literature. This reaction is usually carried out in an alkaline aqueous solution. Side reactions, e.g. hydrolysis of 2-halocarboxylic acid to the respective 2-hydroxycarboxylic acids are also well-known. A conversion of the unreacted amino or hydroxyl groups of the chelating agent intermediates by non-catalyzed alkylation after the incomplete reaction result in a sluggish and incomplete reaction. The alkylation of hydroxyl groups with 2-halocarboxylic acids usually requires strong bases and again, the reactions proceed incompletely.

Lanthanoid-catalyzed Michael additions of hydroxyl groups to maleate are known in the past literature. It has now been surprisingly found that it is possible to convert the free hydroxyl groups in a complex mixture of polycarboxylic acids to the respective carboxymethyl derivatives by a lanthanoid catalyzed alkylation of the hydroxyl group with 2-halocarboxylic acids. Also, the previous efforts of the inventors to convert diethanolamine derivatives to the corresponding O-alkylated carboxymethyl derivatives have failed when 2-haloalkyl carboxylic acids were used in the absence of a lanthanoid catalyst.

The lanthanoid (previously named lanthanide) series comprises the fifteen elements with atomic numbers from 57 to 71. Preferred lanthanoid catalysts are lanthanum (La), praseodymium (Pr), neodymium (Nd), europium (Eu), dysprosium Dy), Erbium (Er) and ytterbium (Yb). The elements of the lanthanoid series may be used in the form of oxides or salts including carbonates, nitrates, chlorides, maleates and octanates.

Residual lanthanide ions/salts are removed from the reaction mixture by methods known in the literature. Such methods can be precipitation as carbonates or oxalates followed by the removal of the precipitate by filtration or centrifugation.

In one embodiment the catalyst is a lanthanoid catalyst including lanthanum(III)oxide and lanthanum(III)salts, such as lanthanum carbonate, lanthanum maleate, lanthanum nitrate, lanthanum chloride or lanthanum octanoate. Michael additions of hydroxyl groups to maleate proceed to some extent without a catalyst. Lanthanoids also catalyze hydroamination of maleates. The reaction time of the addition of maleate for example to ethylenediamine has been shortened from 16 hours to one hour by using lanthanoids as catalysts.

The reactions described here are catalyzed by a lanthanoid catalyst. Thus there is no need to remove or change the catalyst during the process.

In one embodiment the initial molar ratio of the lanthanoid catalyst to diethanol amine is 0.5:1 to 1.5:1. The relatively large amount of catalyst is needed as some of the lanthanide is chelated by the formed products.

In one embodiment 2-halocarboxylic acid or a salt thereof is added after at least 10 mol-% preferably at least 30 mol-%, more preferably at least 50 mol-% of DEA has reacted with maleic acid or a salt thereof. In one embodiment the reaction between maleic acid or a salt thereof and DEA is continued until an equilibrium is reached before adding 2-halocarboxylic acid or a salt thereof in order to ensure the largest possible percentage of chelating agents with at least two succinic acid groups. The progression of the reaction may be monitored using ordinary analytical methods used in organic chemistry, for example gas chromatography (after derivatization), ¹H-NMR spectroscopy and ¹³C-NMR spectroscopy.

In one embodiment the ratio of added 2-halocarboxylic acid or a salt thereof to unreacted hydroxyl and/or amine is 1:1, preferably 1.2:1. Such ratios enable complete conversion of unreacted hydroxyl groups and secondary amines into carboxylic acid groups.

In one embodiment the initial molar ratio of diethanol amine to maleic acid or a salt thereof is 1:1.5 to 1:3.2.

One possible simplified reaction scheme is illustrated in Figure 1. In Figure 1 HA corresponds to a 2-halocaroxylic acid, preferably 2-chloroacetic acid or 2-chloropropionic acid, M corresponds to either H or the counter ion of a salt of the corresponding acid, preferably sodium. The scheme presented in Figure 1 is given only for illustrative purpose and it does not limit the scope of the invention.

A mixture comprising aspartic acid diethoxy succinate (AES) and glycine diethoxy succinate (GES5) is obtained by reacting maleate with diethanol amine under alkaline conditions in the presence of a lanthanoid catalyst to form aspartic acid diethoxy succinate followed by adding 2-haloacetic acid or its salt which reacts with the side-product N-bis- [(1,2-dicarboxy-ethoxy)-ethyl]-amine (BCA4) to form glycine diethoxysuccinate (GES5).

In addition to the above-mentioned reactions, other intermediates present in the reaction mixture, containing hydroxyl groups are reacting with 2-haloacetic acid in the presence of lanthanoid catalyst to form appropriate derivatives. Such derivatives are for example N-[2-(1,2-dicarboxyethoxy)ethyl]-N-[(2-carboxymethoxy)ethyl]glycine (GES4) and N-[2-(1,2-dicarboxyethoxy)ethyl]-N-[2-(carboxymethoxy)ethyl]aspartic acid (AES5).

Respectively a mixture comprising aspartic acid diethoxy succinate and methylglycine diethoxysuccinate is prepared by adding 2-chloropropionic acid or 2-bromopropionic acid, preferably 2-chloropropionic acid to the reaction mixture which reacts with unreacted secondary amines to form methylglycine diethoxysuccinate.

In addition to the above-mentioned reactions, other intermediates present in the reaction mixture, containing hydroxyl groups are reacting with 2-chloropropionic acid or 2-bromopropionic acid, preferably 2-chloropropionic acid to form appropriate derivatives. Such derivatives are for example 2-(2-((2-(1-carboxyethoxy)ethyl)(1-carboxyethyl)amino)ethoxy) succinic acid (MGES 4) and 2-(2-((2-(1-carboxyethoxy)ethyl)(1,2-dicarboxyethyl)amino)ethoxy) succinic acid (AES5b).

The intermediates containing secondary amino groups are reacting with 2-haloacetic acid or 2-halopropionic acid in the presence of lanthanoid catalyst to form polycarbocylic acids capable of forming metal complexes. Such intermediate reacting with 2-haloacetic acid is for example N-bis-[(1,2-dicarboxy-ethoxy)-ethyl]-amine (BCA4), a compound existing in the known AES solution produced by prior art methods and being capable of complexing effectively only earth alkali metals.

With the process described here, BCA4 was reacted completely with 2-chloroacetic acid to produce an effective chelating agent, GES5. According to the experience of the inventors, the conversion of BCA4 to GES5 has not been possible in the absence of lanthanoid catalyst. This conversion is possible by using 2-haloacetic or 2-halopropionic acid in the presence of lanthanoid catalyst.

One embodiment described here is the process for preparation of a mixture of aspartic acid diethoxy succinate and glycine diethoxysuccinate (GES), wherein the process comprises reacting maleate with diethanol amine under alkaline conditions in the presence of a lanthanoid catalyst to form aspartic acid diethoxy succinate. In this reaction, a partially alkylated diethanolamine derivative AES 4 is formed as a side-product. Furthermore, BCA4, a side-product resulting from an anti-Michael reaction of AES6 is formed. BCA4 is converted quantitatively to GES5 by a lanthanoid catalyzed N-alkylation of BCA4. Respectively, the partially reacted intermediates are O-alkylated.

In another embodiment, a mixture comprising aspartic acid diethoxysuccinate and glycine diethoxysuccinate in molar ratios from 10:1 to 1:10 is prepared by addition of 2-haloacetic acid, preferably chloroacetic acid, into the reaction mixture after only a partial reaction of maleate with diethanolamine.

Another embodiment described here relates to the preparation of a mixture comprising aspartic acid diethoxy succinate and methyl glycine diethoxysuccinate (MGES), comprising reacting maleate with diethanol amine under alkaline conditions in the presence of a lanthanoid catalyst to form aspartic acid diethoxy succinate followed by adding 2-halopropionic acid, preferably 2-chloropropionic acid which reacts with unreacted diethanol amine to form methyl glycine diethoxysuccinate (MGES). Respectively, the partially reacted intermediates are O-alkylated.

In another embodiment diethanolamine is reacted with maleate by a catalyzed addition of 2-haloacetic acid by a reaction of aspartic acid or ethylene diamine in order to consume the unreacted maleate from the reaction mixture to produce to the reaction mixture iminodisuccininc acid (IDS) or ethylenediamine disuccinic acid (EDDS), respectively. The conversion of maleate to EDDS or IDS is known.

In one embodiment the amount of the diethanol amine is substoichiometric in relationship to the amount of the maleate. By varying this ratio is possible to obtain a desired amount of GES or MGES in the end product mixture. The molar ratio of diethanol amine to maleic acid or a salt thereof may be between 5:1 and 1:5, preferably between 1:1 and 1:3.

However, it is also possible to use e.g. a stoichiometric amount of diethanol amine in relationship to the amount of the maleate and add chloroacetic acid or chloropropionic acid after or before the maleate addition reaction has reached its equilibrium.

Preferably the maleate and diethanol amine are reacted for a period of time long enough for at least 30 mol-% of the original diethanol amine to be converted into aspartic acid diethoxy succinate. After that 2-halocarboxylic acid is added.

The molar ratio of unreacted diethanol amine and/or intermediates containing hydroxyl groups or secondary amino groups and 2-halocarboxylic acid may be between 10:1 and 1:10, preferably between 1:1 and 1:3.

The initial molar ratio of the lanthanoid catalyst to maleate is preferably between 0.01:2.5 to 1:5, more preferably between 1:3 and 1:6. Expression "initial molar ratio" herein means the ratio when the reaction between diethanol amine and maleate is started.

The initial molar ratio of the lanthanoid catalyst to 2-halocarboxylic acid is preferably between 0.01:2.5 to 1:5, more preferably between 1:3 and 1:6. Expression "initial molar ratio" herein means the ratio when the reaction between unreacted diethanol amine and/or with intermediates containing hydroxyl groups or secondary amino groups and 2-halocarboxylic acid is starter, i.e. when said 2-halocarboxylic acid is added to the reaction mixture.

After completing the reaction, the catalyst is separated using methods known within the field. The catalyst can by separated from the reaction mixture by precipitation as a carbonate by the addition a carbonate salt or carbon dioxide, or as precipitation as an oxalate by the addition of oxalic acid. The formed precipitation can be separated by filtration or centrifugation followed by collecting the supernatant.

The individual components (intermediates or final reaction products) of the mixture are preferably obtained as alkali metal salts or alkaline earth metal salts, but the components may also be obtained in acid form or may be converted from salts into acids.

The present disclosure relates also to a mixture of chelating agents obtained by the process here described.

Further the present disclosure relates to a mixture of chelating agents comprising at least AES6 and GES5 or AES5. In one embodiment the mixture comprises at least 30 % (w/w) of AES6 and at least 2 % (w/w) of AES5 or GES5 or combination of AES5 and GES5. In one embodiment the mixture further comprises at least 3 % (w/w) of AES4.

In one embodiment the mixture comprises less than 1 % (w/w) of DEA, preferably less than 0.5 % (w/w) of DEA, more preferably less than 0.1 % (w/w) of DEA. In one embodiment the mixture is essentially free of DEA.

Table below shows one illustrative composition of the mixture described here.

**Table 1. The concentrations of the ingredients are given as % w/w from the dry matter:**

| Component | % w/w from the dry matter |
|---|---|
| GES5 | 1 to 10 |
| Bicine | 0.5 to 10 |
| *AES4* | 3 to 20 |
| *AES5* | 1 to 10 |
| *AES6* | 30 to 70 |

When oxygen or peroxide compounds are used in bleaching of pulp it is important to remove the transition metals from the fiber before bleaching, since transition metal ions catalyze the decomposition of peroxy compounds, thus forming radical compounds. As a consequence of these reactions the strength properties of the fiber are deteriorated. The decomposition of hydrogen peroxide is catalyzed by transition metals; iron, manganese, and copper are of particular importance in pulp bleaching. The use of chelating agents to remove some of these metal ions from the pulp prior to adding peroxide allows peroxide to be used more efficiently. A chelating agent can be used directly in the bleaching or as a pretreatment before the bleaching proper. This is especially the case when a multistage peroxide bleaching is employed.

The present disclosure relates also to a method of chelating metals by contacting a mixture of chelating agents described here with an aqueous slurry comprising the metals.

The present disclosure relates also to a method of bleaching pulp comprising treating the pulp by a mixture of chelating agents here described or adding the mixture here described to the bleaching state.

The invention is described below with the help of examples. The examples are given only for illustrative purpose and they do not limit the scope of the invention.

### Examples

### Example 1: Reaction of diethanolamine and maleic acid

A solution of maleic acid was prepared by addition of maleic anhydride (75.6 g, 0.77 mol) to deionized water at 55°C. Lanthanum oxide (42.03 g, 0.129 mol) was added to the maleic acid solution and the reaction mixture was heated to 70 °C. The resulting lanthanum-maleate solution was added into a solution of diethanolamine (31.54 g, 0.768 mol). The resulting reaction mixture was stirred at 90°C for 16 hours at pH 8.5-9.5.

Lanthanum catalyst was precipitated out from the reaction mixture by addition of sodium carbonate and the reaction mixture was analyzed as by gas-chromatography after silyl derivatization. The final composition of the reaction mixture is presented in Table 1.

### Example 2 A: Catalyzed reaction of the mixture of Example 1 and 2-haloacetic acid

The reaction product from example 1 was divided into two portions. One portion was heated at 75 °C at pH 9.6 and 2-chloroacetic acid (12.11 g, 0.128 mol) was added over 45 minutes. The pH was adjusted to 8.1 and the reaction mixture was stirred at 90°C for 4 hours.

Lanthanum catalyst was precipitated out from the reaction mixture by addition of sodium carbonate and the reaction mixture was analyzed as by gas-chromatography after silyl derivatization. The composition of the active ingredients of obtained reaction mixture is presented in Table 2.

### Example 2B: Uncatalyzed reaction of mixture of Example 1 and 2-haloacetic acid

The other portion of the reaction product from example 1 was treated in order to precipitate the lanthanum catalyst from the reaction mixture, followed by a treatment of 2-chloroacetic acid as described in example 2A. The reaction mixture was analyzed as by gas-chromatography after silyl derivatization. The final composition of the active ingredients of obtained reaction mixture is presented in Table 2. No conversion of BCA4 to AES5 was obtained. This result clearly shows that the alkylation of the amino group in this reaction product only occurs in the presence of lanthanum catalyst. Furthermore, the O-alkylation of AES4 did not proceed in this reaction in the absence of a lanthanum catalyst.

**Table 2. The concentrations of the active ingredients are given as % w/w from the dry matter:**

| | Example 1 | Example 2B | Example 2A |
|---|---|---|---|
| Maleic acid | 8.85 | 4.79 | 8.85 |
| Diethanolamine | 2.99 | 0.08 | 0.012 |
| GES5 | | 2.74 | |
| Bicine | - | 1.2831 | 2.84 |
| AES4 | 18.87 | 9.79 | 18.92 |
| AES5 | | 3.93 | |
| AES6 | 43.41 | 48.07 | 42.98 |
| BCA4 | 2.84 | 0.00 | 2.85 |

Example 1 discloses the first reaction step of the process described here. As can be seen from the results, a mixture of AES6 and AES4 are the main products with almost 3 % concentration of unreacted diethanolamine. Furthermore, 2.84 % of a poor chelating agent, BCA4, is formed.

Example 2B discloses the second reaction step according to this invention. AES synthesis was run as in Example 1), followed by addition of 2-chloroacetic acid in the presence of Lanthanum catalyst. The total percentage of the effective chelating agent is increased. BCA4 is converted quantitatively to AES5, an effective chelating agent for iron and manganese. Diethanolamine is converted almost quantitatively to bicine and effective chelating agents GES3 and GES5. It should be noted that these reactions are non-optimized reactions. Better conversions could be obtained by optimizing the reaction conditions.

## Claims

1. A process for the preparation of a mixture of chelating agents comprising a compound of Formula (I) wherein at least one of R₁, R₂ and R₃ is a succinic acid group or a salt thereof and at least one of R₁, R₂ and R₃ is a carboxymethyl or 1-carboxyethyl group or a salt thereof 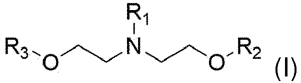 comprising reacting maleic acid or a salt thereof with diethanol amine under alkaline conditions in the presence of a lanthanoid catalyst to form at least one compound having a general formula (I) where at least one of R₁, R₂ and R₃ is a succinic acid group or a salt thereof followed by adding of 2-halocarboxylic acid or a salt thereof which reacts with unreacted diethanol amine and/or with intermediates containing hydroxyl groups or secondary amino groups to form a mixture comprising compounds having a general formula (I) where at least one of R₁, R₂ and R₃ is succinic acid group or a salt thereof and at least one of R₁, R₂ and R₃ is carboxymethyl or 1-carboxyethyl group or a salt thereof.

2. The process of claim 1, wherein unreacted diethanol amine is N-alkylated with a 2-halocarboxylic acid to form a tertiary amine group.

3. The process of any of the preceding claims, wherein the reaction is continued until the reaction mixture comprises
a. at least one compound of Formula (I) selected from N-[2-(1,2-dicarboxyethoxy)ethyl]-N-[2-(carboxymethoxy)ethyl]aspartic acid (AES5) or glycine diethoxy succinate(GES5); and
b. at least one compound Formula (I) wherein R₁, R₂ and R₃ are succinic acid groups (aspartic acid diethyl succinate, AES6); and
c. bis-(2-hydroxyethyl)glycine or bis-(2-hydroxyethyl)methyl glycine.

4. The process of any of the preceding claims, wherein the 2-halocarboxylic acid is bromo- or chloroacetic acid, preferably 2-chloroacetic acid.

5. The process of any of claims 1 - 3, wherein the 2-halocarboxylic acid is a 2-chloro/bromo propionic acid, preferably 2-chloropropionic acid.

6. The process of any of the preceding claims, wherein said catalyst is a lanthanoid catalyst including lanthanum(III)oxide and lanthanum(III)salts, such as lanthanum carbonate, lanthanum maleate, lanthanum nitrate, lanthanum chloride or lanthanum octanoate.

7. The process of any of the preceding claims, wherein 2-halocarboxylic acid or a salt thereof is added after at least 10 mol-% preferably at least 30 mol-%, more preferably at least 50 mol-% of diethanol amine (DEA) has reacted with maleic acid or a salt thereof.

8. The process of any of the preceding claims, wherein the ratio of added 2-halocarboxylic acid or salt thereof to unreacted hydroxyl and/or amine is 1:1, preferably 1.2:1.

9. The process of any of the preceding claims, wherein the reaction between maleic acid or a salt thereof and DEA has reached an equilibrium before adding 2-halocarboxylic acid or a salt thereof.

10. The process of any of the preceding claims, wherein the initial molar ratio of the lanthanoid catalyst to diethanol amine is between 0.5:1 and 1.5:1.

11. The process of any of the preceding claims, wherein the initial molar ratio of diethanol amine to maleic acid or a salt thereof is between 1:1.5 and 1:3.2.

12. A mixture of chelating agents comprising at least 30 % (w/w) of aspartic acid diethyl succinate (AES6) and at least 2 % (w/w) of N-[2-(1,2-dicarboxyethoxy)ethyl]-N-[2-(carboxymethoxy)ethyl]aspartic acid (AES5) or glycine diethoxy succinate (GES5) or combination of AES5 and GES5.

13. The mixture of claim 12, wherein said mixture contains less than 1 % (w/w) of DEA.

14. Method of chelating metals by contacting the mixture of claim 12 or 13 with aqueous slurry comprising the metals.

15. Method of bleaching pulp comprising treating the pulp by the mixture of claim 12 or 13 or adding the mixture of claim 12 or 13 to the bleaching state.

## Patentansprüche

1. Verfahren zum Herstellen einer Mischung aus Chelatbildnern, umfassend eine Verbindung der Formel (I) wobei mindestens einer der Reste R₁, R₂ und R₃ eine Bernsteinsäuregruppe oder ein Salz davon ist und mindestens einer der Reste R₁, R₂ und R₃ eine Carboxymethyl- oder 1-Carboxyethylgruppe oder ein Salz davon ist, 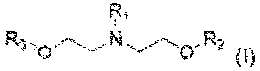 umfassend das Umsetzen von Maleinsäure oder eines Salzes davon mit Diethanolamin unter alkalischen Bedingungen in Gegenwart eines Lanthanoid-Katalysators zum Bilden mindestens einer Verbindung der allgemeinen Formel (I), wobei mindestens einer der Reste R₁, R₂ und R₃ eine Bernsteinsäuregruppe oder ein Salz davon ist, gefolgt von der Zugabe von 2-Halogencarbonsäure oder einem Salz davon, die mit nicht umgesetztem Diethanolamin und/oder mit Zwischenprodukten, die Hydroxylgruppen oder sekundäre Aminogruppen enthalten, reagiert, um eine Mischung zu bilden, die Verbindungen mit der allgemeinen Formel (I) umfasst, wobei mindestens einer der Reste R₁, R₂ und R₃ eine Bernsteinsäuregruppe oder ein Salz davon ist und mindestens einer der Reste R₁, R₂ und R₃ eine Carboxymethyl- oder 1-Carboxyethylgruppe oder ein Salz davon ist.

2. Verfahren nach Anspruch 1, wobei nicht umgesetztes Diethanolamin mit einer 2-Halogencarbonsäure unter Bildung einer tertiären Aminogruppe N-alkyliert wird.

3. Verfahren nach einem der vorhergehenden Ansprüche, wobei die Reaktion fortgesetzt wird, bis die Reaktionsmischung Folgendes umfasst:
a. mindestens eine Verbindung der Formel (I), die aus N-[2-(1,2-Dicarboxyethoxy)ethyl]-N-[2-(Carboxymethoxy)ethyl]asparaginsäure (AES5) oder Glycindiethoxysuccinat (GES5) ausgewählt ist; und
b. mindestens eine Verbindung der Formel (I) wobei R₁, R₂ und R₃ Bernsteinsäuregruppen (Asparaginsäurediethylsuccinat, AES6); und
c. Bis-(2-hydroxyethyl)glycin oder Bis-(2-hydroxyethyl)methylglycin sind.

4. Verfahren nach einem der vorhergehenden Ansprüche, wobei die 2-Halogencarbonsäure Brom- oder Chloressigsäure, bevorzugt 2-Chloressigsäure, ist.

5. Verfahren nach einem der Ansprüche 1 bis 3, wobei die 2-Halogencarbonsäure eine 2-Chlor-/Brompropionsäure, bevorzugt 2-Chlorpropionsäure ist.

6. Verfahren nach einem der vorhergehenden Ansprüche, wobei der Katalysator ein Lanthanoid-Katalysator ist, der Lanthan(III)oxid und Lanthan(III)salze, wie Lanthancarbonat, Lanthanmaleat, Lanthannitrat, Lanthanchlorid oder Lanthanoctanoat, enthält.

7. Verfahren nach einem der vorhergehenden Ansprüche, wobei 2-Halogencarbonsäure oder ein Salz davon zugegeben wird, nachdem mindestens 10 mol-%, bevorzugt mindestens 30 mol-%, stärker bevorzugt mindestens 50 mol-% Diethanolamin (DEA) mit Maleinsäure oder einem Salz davon reagiert haben.

8. Verfahren nach einem der vorhergehenden Ansprüche, wobei das Verhältnis von zugegebener 2-Halogencarbonsäure oder dem Salz davon zu nicht umgesetztem Hydroxyl und/oder Amin 1: 1, bevorzugt 1,2: 1 beträgt.

9. Verfahren nach einem der vorhergehenden Ansprüche, wobei die Reaktion zwischen Maleinsäure oder einem Salz davon und DEA ein Gleichgewicht erreicht hat, bevor 2-Halogencarbonsäure oder ein Salz davon zugegeben wird.

10. Verfahren nach einem der vorhergehenden Ansprüche, wobei das anfängliche Molverhältnis des Lanthanoidkatalysators zu Diethanolamin zwischen 0,5: 1 und 1,5: 1 liegt.

11. Verfahren nach einem der vorhergehenden Ansprüche, wobei das anfängliche Molverhältnis von Diethanolamin zu Maleinsäure oder einem Salz davon zwischen 1: 1,5 und 1: 3,2 liegt.

12. Mischung von Chelatbildnern, die mindestens 30 % (w/w) Asparaginsäurediethylsuccinat (AES6) und mindestens 2 % (w/w) N-[2-(1,2-Dicarboxyethoxy)ethyl]-N-[2-(Carboxymethoxy)ethyl]asparaginsäure (AES5) oder Glycindiethoxysuccinat (GES5) oder eine Kombination aus AES5 und GES5 umfasst.

13. Mischung nach Anspruch 12, wobei die Mischung weniger als 1 % (w/w) DEA enthält.

14. Verfahren zum Chelatisieren von Metallen durch Inkontaktbringen der Mischung nach Anspruch 12 oder 13 mit einer wässrigen Aufschlämmung, welche die Metalle umfasst.

15. Verfahren zum Bleichen von Zellstoff, bei dem der Zellstoff mit der Mischung nach Anspruch 12 oder 13 behandelt oder die Mischung nach Anspruch 12 oder 13 dem Bleichzustand zugegeben wird.

## Revendications

1. Procédé pour la préparation d'un mélange d'agents chélatants comprenant un composé de Formule (I), au moins l'un parmi R₁, R₂ et R₃ étant un groupe acide succinique ou un sel correspondant et au moins l'un parmi R₁, R₂ et R₃ étant un groupe carboxyméthyle ou 1-carboxyéthyle ou un sel correspondant 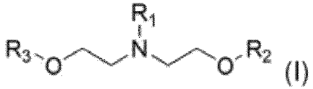 comprenant la mise en réaction d'acide maléique ou d'un sel correspondant avec de la diéthanolamine dans des conditions alcalines en la présence d'un catalyseur de type lanthanoïde pour former au moins un composé possédant une formule générale (I) où au moins l'un parmi R₁, R₂ et R₃ est un groupe acide succinique ou un sel correspondant suivie par l'ajout d'un acide 2-halogénocarboxylique ou d'un sel correspondant qui réagit avec de la diéthanolamine qui n'a pas réagi et/ou avec des intermédiaires contenant des groupes hydroxyle ou des groupes amino secondaire pour former un mélange comprenant des composés possédant une formule générale (I) où au moins l'un parmi R₁, R₂ et R₃ est un groupe acide succinique ou un sel correspondant et au moins l'un parmi R₁, R₂ et R₃ est un groupe carboxyméthyle ou 1-carboxyéthyle ou un sel correspondant.

2. Procédé selon la revendication 1, la diéthanolamine qui n'a pas réagi étant N-alkylée avec un acide 2-halogénocarboxylique pour former un groupe amine tertiaire.

3. Procédé selon l'une quelconque des revendications précédentes, la réaction étant poursuivie jusqu'à ce que le mélange réactionnel comprenne
a. au moins un composé de Formule (I) choisi parmi l'acide N-[2-(1,2-dicarboxyéthoxy)éthyl]-N-[2-(carboxyméthoxy)éthyl]aspartique (AES5) et le glycine-diéthoxy-succinate (GES5) ; et
b. au moins un composé de Formule (I), R₁, R₂ et R₃ étant des groupes acide succinique (acide aspartique-diéthyl-succinate, AES6) ; et
c. la bis-(2-hydroxyéthyl)glycine ou la bis-(2-hydroxyéthyl)méthyl-glycine.

4. Procédé selon l'une quelconque des revendications précédentes, l'acide 2-halogénocarboxylique étant l'acide bromoacétique ou l'acide chloroacétique, préférablement l'acide 2-chloroacétique.

5. Procédé selon l'une quelconque des revendications 1 à 3, l'acide 2-halogénocarboxylique étant un acide 2-chloropropionique ou un acide 2-bromopropionique, préférablement l'acide 2-chloropropionique.

6. Procédé selon l'une quelconque des revendications précédentes, ledit catalyseur étant un catalyseur de type lanthanoïde y compris l'oxyde de lanthane(111) et des sels de lanthane(111), tels que le carbonate de lanthane, le maléate de lanthane, le nitrate de lanthane, le chlorure de lanthane ou l'octanoate de lanthane.

7. Procédé selon l'une quelconque des revendications précédentes, l'acide 2-halogénocarboxylique ou un sel correspondant étant ajouté après qu'au moins 10 % en moles préférablement au moins 30 % en moles, plus préférablement au moins 50 % en moles de diéthanolamine (DEA) a réagi avec l'acide maléique ou un sel correspondant.

8. Procédé selon l'une quelconque des revendications précédentes, le rapport d'acide 2-halogénocarboxylique ou de sel correspondant ajouté sur hydroxyle et/ou amine n'ayant pas réagi(e) étant de 1:1, préférablement de 1,2:1.

9. Procédé selon l'une quelconque des revendications précédentes, la réaction entre l'acide maléique ou un sel correspondant et la DEA ayant atteint un équilibre avant l'ajout d'acide 2-halogénocarboxylique ou d'un sel correspondant.

10. Procédé selon l'une quelconque des revendications précédentes, le rapport molaire initial du catalyseur de type lanthanoïde sur la diéthanolamine étant compris entre 0,5:1 et 1,5:1.

11. Procédé selon l'une quelconque des revendications précédentes, le rapport molaire initial de la diéthanolamine sur l'acide maléique ou un sel correspondant étant compris entre 1:1,5 et 1:3,2.

12. Mélange d'agents chélatants comprenant au moins 30 % (p/p) d'acide aspartique-diéthyl-succinate (AES6) et au moins 2 % (p/p) d'acide N-[2-(1,2-dicarboxyéthoxy)éthyl]-N-[2-(carboxyméthoxy)éthyl]aspartique (AES5) ou de glycine-diéthoxy-succinate (GES5) ou d'une combinaison de AES5 et de GES5.

13. Mélange selon la revendication 12, ledit mélange contenant moins de 1 % (p/p) de DEA.

14. Procédé de chélation de métaux par mise en contact du mélange selon la revendication 12 ou 13 avec une suspension aqueuse comprenant les métaux.

15. Procédé de blanchiment de pulpe comprenant le traitement de la pulpe par le mélange selon la revendication 12 ou 13 ou l'ajout du mélange selon la revendication 12 ou 13 à l'état de blanchiment.
